(19) Europäisches Patentamt
European Patent Office
Office européen des brevets

(11) Publication number: **0 044 583**
**B1**

(12) **EUROPEAN PATENT SPECIFICATION**

(45) Date of publication of patent specification: **01.02.84**

(51) Int. Cl.³: **B 01 J 23/24,** B 01 J 27/26

(21) Application number: **81200737.5**

(22) Date of filing: **30.06.81**

(54) **Process for the preparation of supported molybdenum/tungsten compositions and their use for the isomerization and disproportionation of olefins.**

(30) Priority: **21.07.80 US 170352**

(43) Date of publication of application:
**27.01.82 Bulletin 82/4**

(45) Publication of the grant of the patent:
**01.02.84 Bulletin 84/5**

(84) Designated Contracting States:
**BE DE FR GB IT NL**

(56) References cited:
**DE - A - 2 217 675**
**FR - A - 2 256 255**
**US - A - 3 418 390**

**IND. & ENGEN. CHEM. PRODUCT RESEARCH AND DEVELOPMENT vol. 3, pages 170-173, (1964) R.L. BANKS: "Olefin disproportionation"**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Hoxmeier, Ronald James**
**2823 Waypark**
**Houston Texas 77082 (US)**

(74) Representative: **Puister, Antonius Tonnis, Mr. et al,**
**P.O. Box 302**
**NL-2501 CH The Hague (NL)**

**EP 0 044 583 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation of supported molybdenum/tungsten compositions and their use for the isomerization and disproportionation of olefins

The invention relates to a process for the preparation of compositions containing supported molybdenum and/or supported tungsten. These compositions are particularly useful for the simultaneous isomerization and disproportionation of olefins. The invention, therefore, also relates to a process for the isomerization and disproportionation of olefins in the presence of these compositions. By "isomerization" is meant shifting of the carbon-carbon double bond in an olefin without change of the carbon skeleton.

Transition metal carbonyl complexes have long been known for their catalytic activity. For example, compounds of the general formula:

$$R[M(CO)_5CN]$$

wherein R represents a monovalent cation and M a molybdenum or tungsten atom, are known from U.S. patent specification 3,686,136 as catalysts for the disproportionation of olefins. However, the compounds of this general formula are not catalysts for the isomerization of olefins.

The transition metals also form complexes with the cyanide ion which is iso-electronic with carbon monoxide. Unlike metal carbonyls, metal cyanides have an unusually high thermal stability. This would allow these cyanides to operate in temperature ranges which are too high for metal carbonyls. A number of Group VIII metal cyanide complexes are known to catalyze certain organic reactions. The indication "Group VIII" refers to the Periodic Table of the Elements shown on the inside cover of "Handbook of Chemistry and Physics", 59th edition (1978—1979). One example of these cyanide complexes is the trivalent anion $HCo(CN)_5^{3-}$ which is a catalyst for the selective hydrogenation of conjugated diolefins to mono-olefins. Other examples of these cyanide complexes are potassium octacyanomoybdate(IV) with the formula $K_4Mo(CN)_8$ and potassium octacyanotungstate(IV) with the formula $K_4W(CN)_8$. However, these two complexes have not been reported to possess any catalytic properties, though they have been known since the thirties.

Applicant has now discovered a method utilizing the alkali metal octacyanomolybdates and -tungstates to prepare molybdenum- and tungsten-containing compositions which are catalysts for the isomerization and disproportionation of olefins.

The invention provides a process for the preparation of a molybdenum- and/or tungsten-containing composition, characterized by impregnating a porous, inert support with an aqueous solution containing octacyanomolybdic acid —$H_4Mo(CN)_8$—, octacyanotungstic acid —$H_4W(CN)_8$— or a mixture thereof, drying the impregnated support and calcining the impregnated support in a non-oxidizing environment.

The impregnated support is preferably calcined at a temperature in the range of from 400°C to 600°C.

The supports utilized to prepare the composition in accordance with this invention must be sufficiently porous to allow impregnation with aqueous solutions of the said cyano acids. The minimum porosity necessary can readily be determined by routine experimentation. The supports must be inert to the preparation techniques used, i.e. they must not decompose, deteriorate or degrade under the impregnation, drying or calcining conditions. If the compositions are being prepared for use in specific catalytic reactions, then the supports should also be inert to the reaction conditions. Examples of suitable supports are carbon, alumina, clay, silica, pumice, magnesia, alumino-silica, zirconia, titania, etc. Preferred supports are aluminas, silicas and mixtures thereof, because they give particularly good results in the isomerization and disproportionation of olefins. The amount of molybdenum and/or tungsten present in the final composition is not critical, but normally will range from 0.1 to 25%, preferably from about 1 to about 10% by weight of the total composition weight.

The $H_4Mo(CN)_8$ and $H_4W(CN)_8$ present in the impregnating solutions are typically prepared by ion exchanging an alkali metal cyanide $M_4Mo(CN)_8$, wherein M represents a Li, Na, K, Rb or Cs atom, with hydrogen ion using the acid form of strong acid ion-exchange resins. Typical useful resins comprise the sulphonated phenolic and styrenic types. The macroreticular sulphonated styrene-divinylbenzene resins are particularly useful.

Impregnation of the supports with aqueous solutions of $H_4Mo(CN)_8$ and $H_4W(CN)_8$ is routine. A preferred technique is the so-called dry-impregnation technique in which only that amount of aqueous solution is used which can readily be absorbed by the support.

After impregnation the support is dried to remove free water. Drying is accomplished in a routine fashion, as for example, passing dry nitrogen or helium or other inert gas over the impregnated support, or by the use of sub-atmospheric pressure. The use of an oxidizing environment should be avoided in order to prevent oxidation at the molybdenum or tungsten. The drying step can be combined with the calcining step.

After drying, the impregnated support is calcined in a non-oxidizing, suitably a neutral environment, preferably at a temperature in the range of from 400 to 600°C. Times of calcining are not critical and are typically in the range of from 0.1 to 50 hours; lower calcining temperatures requiring

longer times and vice versa. Suitable examples of a neutral environment are nitrogen, helium, argon, etc. Nitrogen is preferred. The calcination may be carried out at sub-atmospheric pressure.

The compositions prepared according to this invention and their use as catalysts will be further described by the following Examples.

Example 1

*Preparation of a composition containing molybdenum on alumina*

An amount of $K_4Mo(CN)_8$ was prepared according to the synthesis reported in *Inorganic Synthesis,* Vol. 3, pp. 160—162. $K_4Mo(CN)_8$ (6 g, 10 mmol.) was dissolved in water (10 ml) and the solution obtained was passed down a strong acid ion-exchange column (containing a sulphonated styrene-divinylbenzene resin; Amberlite® IR—120 HCP, Rohm & Haas Co.). An amount of 50 ml of the solution collected at the bottom of the column was pulled to dryness under sub-atmospheric pressure. The $H_4Mo(CN)_8$ thus obtained was dissolved in water (10 ml) and the solution obtained was used to impregnate 20 ml of alumina. The resulting material was dried at sub-atmospheric pressure in an oven overnight at 275°C. Five cc of this dried material were loaded in a glass reactor tube under nitrogen and thermally activated under a stream of nitrogen at 500°C for 2 hours.

Example 2

*Preparation of a composition containing molybdenum on alumina (base treated)*

Kaiser® A—201 $Al_2O_3$ (manufactured by Kaiser Chemicals, Louisiana, specific surface area 335 $m^2/g$, pore volume 0.51 ml/g, loss on ignition 6.0%, sodium content 0.35%w, calculated as $Na_2O$) was refluxed for one hour in a 5% aqueous KOH solution. The resulting support was then washed liberally with water until the wash solutions had a pH of 7. The support was calcined two hours at 500°C in air.

Then $K_4Mo(CH)_8$ (7 g) was dissolved in water (10 ml) and the solution obtained was passed through a strong acid ion-exchange column (Amberlite IR—120 HCP, Rohm & Haas Co., molar ratio $H^+$ resin/$K^+$ compound = 3/1). A 50 ml portion of eluent was collected and reduced to dryness yielding 4.6 g of solid.

An amount (2 g) of this solid was dissolved in water (5 ml) and the solution was used to dry impregnate 10 cc of the above base-treated Kaiser A—201 $Al_2O_3$. The impregnated alumina was dried at sub-atmospheric pressure in an oven at 50°C overnight. The dried material (3 ml) was placed in a glass reactor tube under nitrogen and heated at 200°C for 30 minutes, 350°C for 30 minutes, then 500°C for 1 hour.

Example 3

*Preparation of tungsten on alumina*

An amount of $K_4W(CN)_8$ was prepared according to the synthesis reported in *Inorganic Synthesis,* Vol. 7, pp. 142—146 utilizing granular tin. $K_4W(CN)_8$ (6 g) was dissolved in water (10 ml) and the solution obtained was passed down a strong acid ion exchange column (Amberlyst® IR—120 HCP). An amount of 50 ml of the solution collected at the bottom of the column was pulled to dryness under sub-atmospheric pressure. The $H_4W(CN)_8$ thus obtained was dissolved in water (10 ml) and the solution obtained was used to impregnate 20 ml of alumina. The resulting material was dried at sub-atmospheric pressure overnight at 275°C and then thermally activated in a stream of nitrogen at 500°C for 2 hours.

Example 4

*Preparation of tungsten on silica and of molybdenum on silica*

Techniques similar to those described above were used to prepare a composition containing molybdenum supported on silica and one containing tungsten supported on silica (Davison® Grade 57 silica gel (Grace G.m.b.H., Werk Bad Homburg, Germany)). This silica gel had a specific surface area of 325 $m^2/g$ and a pore volume of 1.16 ml/g.

Example 5

*Preparation of tungsten on carbon and of molybdenum on carbon*

Techniques similar to those described above were used to prepare a composition containing molybdenum supported on active carbon and one containing tungsten supported on active carbon in the form of pellets (obtained from Union Carbide and prepared by pyrolysis of polystyrene).

Example 6

*Use of molybdenum on alumina*

The Mo/alumina composition of Example 1 was tested with 1-decene, as a catalyst for isomerization and disproportionation. A glass reactor tube was loaded with the composition (5 $cm^3$). The loaded tube was heated to 150°C and 1-decene was fed to the reactor at a liquid hourly space velocity (LHSV) of 3.6 l/l.h by means of a sage syringe pump (Model No. 255—2) using a 25 ml glass syringe. Product was collected in glass vials immersed in an ice bath. Analyses by means of gas chromatography (GC) showed a 45% conversion of feed with the product containing every olefin from

$C_2^=$ through $C_{18}^=$ plus small amounts of some olefins having more than 18 carbon atoms per molecule. Mass spectroscopy-GC analyses confirmed with products to be the above-mentioned olefins and these results are shown in the Table. It is apparent that this composition iosomerizes and dispropitonates the olefin feed simultaneously.

Example 7
*Use of molybdenum on alumina (base treated)*

To suppress any contribution that the acid character of the alumina suipport might contribute to the isomerization of 1-decene, a molybdenum/alumina composition was prepared using based-neutralized alumina (Example 2 above). This composition was tested with 1-decene, as a catalyst for isomerization and disproportionation, using the process described above (LHSV = 2). GC analysis of the product indicates greater than 90% conversion with extensive isomerization activity. These results are shown in the Table.

Example 8
*Use of tungsten on alumina*

Testing the tungsten/alumina composition of Example 3 under the same conditions that were used for testing the composition of Example 1 gave a conversion of 65% of 1-decene. The results of the GC spectra are shown in the Table.

Example 9
*Use of tungsten on silica*

Testing the tungsten/silica composition of Example 4 under the same conditions that were used for testing the composition of Example 3 gave similar results but lower conversion.

Example 10
*Use of molybdenum on carbon*

Testing of the molybdenum/carbon showed that this material isomerized 1-decene and had no disproportionation activity.

Comparative Experiment

A conventional catalyst containing 5% by weight of CoO and 12% by weight of $MoO_3$, supported on alumina was prepared and heated at 525°C for 16 hours in a stream of nitrogen. Then, the experiment described in Example 7 was modified by using the conventional catalyst instead of the catalyst used in Example 7. The results of the modified experiment are shown in the Table. A comparison between Example 8 and the Comparative Experiment shows that the catalyst prepared according to the invention is considerably more active than the conventional catalyst.

TABLE

| Olefins in product | Example 6 %w | Example 7 %w | Example 8 %w | Comparative experiment %w |
|---|---|---|---|---|
| $C_4$ | 0.03 | – | – | 0.1 |
| $C_5$ | 0.03 | 0.4 | – | 0.2 |
| $C_6$ | 0.04 | 1.3 | 0.3 | 0.3 |
| $C_7$ | 0.06 | 2.1 | 0.9 | 0.6 |
| $C_8$ | 1.1 | 4.1 | 1.8 | 1.3 |
| $C_9$ | 4.1 | 3.0 | 7.0 | 6.0 |
| $C_{10}$ | 67.5 | 18.0 | 53.3 | 18.0 |
| $C_{11}$ | 4.9 | 8.6 | 6.5 | 7.5 |
| $C_{12}$ | 0.7 | 8.9 | 8.8 | 1.4 |
| $C_{13}$ | 0.3 | 12.6 | 5.7 | 0.6 |
| $C_{14}$ | 0.7 | 9.4 | 3.3 | 0.8 |
| $C_{15}$ | 1.7 | 7.6 | 5.3 | 1.8 |
| $C_{16}$ | 4.2 | 7.4 | 2.8 | 7.6 |
| $C_{17}$ | 6.8 | 5.3 | 1.5 | 16.0 |
| $C_{18}$ | 7.9 | 4.8 | 1.7 | 32.7 |
| $C_{19}$ | – | 2.5 | 0.8 | 2.6 |
| $C_{20}$ | – | 1.6 | 0.3 | 1.6 |
| $C_{21}$ | – | 1.1 | – | 0.5 |
| $C_{22}$ | – | 0.7 | – | 0.5 |
| $C_{23}$ | – | 0.5 | – | – |

## Claims

1. A process for the preparation of a molybdenum- and/or tungsten-containing composition characterized by impregnating a porous, inert support with an aqueous solution containing octacyanomolybdic acid —$H_4Mo(CN)_8$—, octacyanotungstic acid —$H_4W(CN)_8$— or a mixture thereof, drying the impregnated support and calcining the impregnated support in a non-oxidizing environment.

2. A process as claimed in claim 1 characterized in that the composition has a molybdenum and/or tungsten content in the range of from 0.1 to 25% by weight, calculated on the total weight of the composition.

3. A process as claimed in claim 1 or 2, characterized in that the support is alumina, silica or a mixture thereof.

4. A process as claimed in claim 1 or 3, characterized in that the $H_4Mo(CN)_8$, $H_4W(CN)_8$ or a mixture thereof has been prepared by ion-exchanging an alkali metal cyanide of the general formula $M_4Mo(CN)_8$ (wherein M represents a Li, Na, K, Rb or Cs atom) with the hydrogen form of a strong acid ion-exchange resin.

5. A process as claimed in claim 4, characterized in that the ion exchange resin is a sulphonated styrene-divinylbenzene resin.

6. A process as claimed in any one of claims 1 to 5, characterized in that the support is calcined in a nitrogen environment.

7. A process as claimed in any one of claims 1 to 6, characterized in that the impregnated support has been calcined at a temperature in the range from 400 to 600°C.

8. Use of a composition as directly obtained by a process according to any one of claims 1 to 7 in a process for the simultaneous isomerization and disproportionation of olefins.

**Patentansprüche**

1. Verfahren zur Herstellung einer Molybdän- und/oder Wolfram-enthaltenden Masse, dadurch gekennzeichnet, daß ein poröser, inerter Träger mit einer wässrigen Lösung, enthaltend Octacyanmolybdänsäure $H_4Mo(CN)_8$, Octacyanwolframsäure $H_4W(CN)_8$ oder deren Gemische imprägniert, der imprägnierte Träger getrocknet und der trockene imprägnierte Träger in nichtoxidierendem Milieu gebrannt wird.

2. Verfahren nach Anspruch 1, dadurch gekennzeichnet, daß man eine Masse herstellt, in welcher Molybdän und/oder Wolfram in einer Menge von 0,1 bis 25 Gew.-%, bezogen auf Gesamtgewicht der Masse, enthalten ist (sind).

3. Verfahren nach Anspruch 1 oder 2, dadurch gekennzeichnet, daß man als Träger Aluminiumoxid, Siliciumdioxid oder deren Gemisch verwendet.

4. Verfahren nach Anspruch 1 oder 3, dadurch gekennzeichnet, daß $H_4Mo(CN)_8$ und/oder $H_4W(CN)_8$ hergestellt worden sind durch Ionenaustausch eines Alkalicyanids der allgemeinen Formel $M_4Mo(CN)_8$ bzw. $M_4W(CN)_8$ mit der Wasserstofform eines stark sauren Ionenaustauscherharzes, wobei M, Li, Na, K, Rb oder Cs ist.

5. Verfahren nach Anspruch 4, dadurch gekennzeichnet, daß man als Ionenaustauscherharz ein sulfoniertes Styrol/Divinylbenzol-Harz verwendet.

6. Verfahren nach Anspruch 1 bis 5, dadurch gekennzeichnet, daß man in Stickstoff-atmosphäre brennt.

7. Verfahren nach Anspruch 1 bis 6, dadurch gekennzeichnet, daß man bei einer Temperatur von 400 bis 600°C brennt.

8. Anwendung der Masse, erhalten nach dem Verfahren der Ansprüche 1 bis 7, für ein Verfahren zur gleichzeitigen Isomerisierung und Disproportionierung von Olefinen.

**Revendications**

1. Un procédé pour la préparation d'une composition contenant du molybdène et/ou du tungstène caractérisé par l'imprégnation d'un support poreux, inerte avec une solution aqueuse contenant de l'acide octacyanomolybdique $H_4Mo(CN)_8$—, de l'acide octacyanotungstique —$H_4W(CN)_8$— ou un mélange de ceux-ci, le séchage du support imprégné et la calcination du support imprégné dans un milieu non oxydant.

2. Un procédé selon la revendication 1, caractérisé en ce que la composition possède une teneur en molybdène et/ou en tungstène comprise entre 0,1 et 25% en poids, calculée sur le poids total de la composition.

3. Un procédé selon la revendication 1 ou 2, caractérisé en ce que le support est de l'alumine, de la silice ou un mélange de ceux-ci.

4. Un procédé selon la revendication 1 ou 3, caractérisé en ce que du $H_4Mo(CN)_8$, du $H_4W(CN)_8$ ou un mélange de ceux-ci a été préparé par échange d'ion entre un cyanure de métal alcalin de formule générale $M_4Mo(CN)_8$ (où M représente un atome Li, Na, K, Rb ou Cs) et la forme d'hydrogène d'une résine échangeuse d'ion acide forte.

5. Un procédé selon la revendication 4, caractérisé en ce que la résine échangeuse d'ion est une résine divinylbenzène-styrène sulfonée.

6. Un procédé selon n'importe laquelle des revendications 1 à 5, caractérisé en ce que le support est calciné dans un milieu d'azote.

7. Un procédé selon n'importe laquelle des revendications 1 à 6, caractérisé en ce que le support imprégné a été calciné à une température comprise entre 400 et 600°C.

8. L'utilisation d'une composition obtenue directement par un procédé selon n'importe laquelle des revendications 1 à 7 dans un procédé pour l'isomérisation et la disproportion simultanée d'oléfines.